# EUROPEAN PATENT APPLICATION

(11) **EP 4 732 813 A1**
(43) Date of publication of application: **29.04.2026**
(21) Application number: 24825483.1
(22) Date of filing: 22.01.2024
(51) Int. Cl.: A61F 7/10, B65D 30/02, B65D 65/02

(54) **FLUID-SEALING CONTAINER**

(30) Priority: 23.06.2023 JP 2023102974
(71) Applicant: KAO CORPORATION, Tokyo 103-8210 (JP)
(72) Inventor: TANAKA, Masahito, Tokyo 103-8210 (JP); ISHII, Tomomi, Tokyo 103-8210 (JP)
(74) Representative: Hoffmann Eitle
(86) International application number: PCT/JP2024/001715
(87) International publication number: WO 2024/262071

(57) **Abstract**

A fluid sealed container includes a main body part formed of a film made of a thermoplastic elastomer or a thermoplastic resin, and an injection port part that is connected to the main body part and is configured to be openable and closable to be able to hermetically seal the main body part, in which the film forming the main body part is water-impermeable, has a glass transition temperature of less than 0 degrees centigrade, and has a carbon dioxide gas penetration of equal to or more than 5 L/(m²·24 hr·atm) as measured under the condition of 10 degrees centigrade in accordance with JIS K 7126-2 Annex B, and further has a tensile strength of equal to or more than 8 N/10 mm as measured under the condition of 10 degrees centigrade in accordance with JIS K 7161-1.

## Description

### TECHNICAL FIELD

The present invention relates to a fluid sealed container that can be used as a human body cooling tool by enclosing carbon dioxide gas-containing cold water.

### BACKGROUND ART

Products for cooling each portion of a human body have been conventionally widely used. As such products, known are, for example, a cooling gel sheet, and a cooling tool in which a cooling agent, water, or the like is hermetically sealed and packaged. Furthermore, also known is a cooling tool which can be filled with carbon dioxide (carbon dioxide gas, dry ice, or the like) in addition to or instead of water or the like.

For example, PATENT DOCUMENT 1 discloses a cooling bag that can be filled with dry ice, and has one surface in which a large number of pores through which carbon dioxide gas can pass are formed, and another surface formed of a thermal insulating material through which the carbon dioxide gas cannot pass.

### CITATION LIST

PATENT DOCUMENT 1: Japanese Patent Laid-Open No. 2008-023274

### SUMMARY OF THE INVENTION

According to one aspect of the present invention, there is provided a fluid sealed container that includes a main body part formed of a film made of a thermoplastic elastomer or a thermoplastic resin, and an injection port part that is connected to the main body part and is configured to be openable and closable to be able to hermetically seal the main body part, in which the film forming the main body part is water-impermeable, has a glass transition temperature of less than 0 degrees centigrade, and has a carbon dioxide gas penetration of equal to or more than 5 L/(m²·24 hr·atm) as measured under the condition of 10 degrees centigrade in accordance with JIS K 7126-2 Annex B, and further has a tensile strength of equal to or more than 8 N/10 mm as measured under the condition of 10 degrees centigrade in accordance with JIS K 7161-1.

### BRIEF DESCRIPTION OF THE DRAWINGS

[Fig. 1] Fig. 1 is a perspective view of a fluid sealed container according to the present embodiment.
[Fig. 2] Fig. 2 is a front view showing a cooling tool obtained by enclosing carbon dioxide gas-containing cold water in the fluid sealed container according to the present embodiment.
[Fig. 3] Fig. 3 is a schematic diagram of an example in which a cooling tool obtained by enclosing carbon dioxide gas-containing cold water in the fluid sealed container according to the present embodiment is used by being worn around a leg.
[Fig. 4] Fig. 4 is a schematic diagram of an example in which the cooling tool in Fig. 2 is worn around a neck.

### DESCRIPTION OF EMBODIMENTS

When each portion of a human body is cooled using a cooling tool to quickly reduce the temperature (up to equal to or less than 15 degrees centigrade), the blood flow in the portion is deteriorated, so that there is a case where the alleviation effect on fatigue or swelling cannot be sufficiently obtained. In addition, the cooling effect is likely to be local. Therefore, it is desirable to allow carbon dioxide gas to penetrate and permeate from the cooling tool together with performing this cooling at the same time so that the cooling action and the action of the carbon dioxide gas (action of maintaining or promoting blood circulation) are exerted. However, in a case where the carbon dioxide gas-containing cold water is enclosed in a container to use the container as the cooling tool, it is difficult for the container to achieve both of the followability (fit feeling) to the human body at a low temperature and the durability (resistance to generation of breakage or crack) at the low temperature if the carbon dioxide gas penetration is to be maintained at the low temperature.

According to the present invention, there can be provided a fluid sealed container that is excellent in both of followability to a human body at a low temperature and durability at the low temperature while maintaining carbon dioxide gas penetration at the low temperature, and in which carbon dioxide gas-containing cold water of equal to or less than 15 degrees centigrade is enclosed to be usable as a human body cooling tool. The carbon dioxide gas-containing cold water of equal to or less than 15 degrees centigrade is enclosed in the fluid sealed container to serve as the cooling tool, making it possible for the cooling tool to perform cooling and alleviate fatigue and the like without deteriorating fit feeling at a contact portion and a blood flow in the contact portion, so that the cooling effect is easily obtained in a wide range of the human body and breakage or crack is unlikely to be generated during its use or the like.

A preferred embodiment of the present invention will be described below with reference to the drawings. In all the drawings, similar constituent components are denoted by the same reference numerals, and the duplicated description will not be repeated. In some of the drawings, for the sake of convenience, there are parts not indicated by reference numerals (reference numerals are not used). Furthermore, the dimensional proportions of each member shown in the drawings may differ from the actual dimensional proportions in order to facilitate understanding of the invention.

### [Overall Configuration]

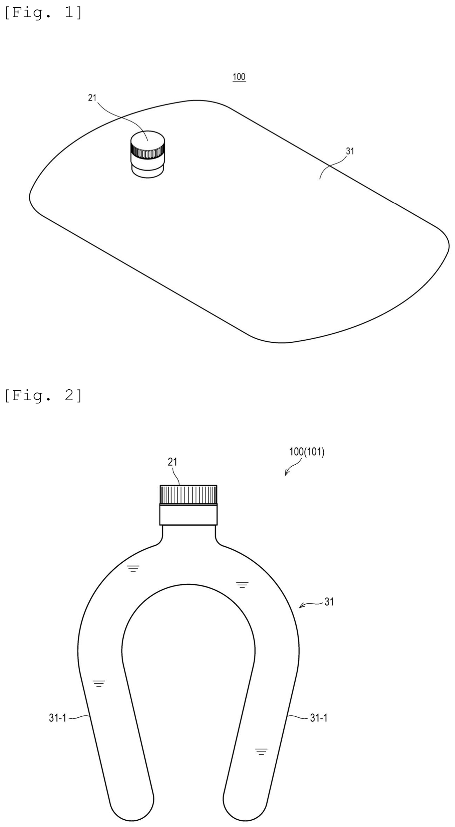

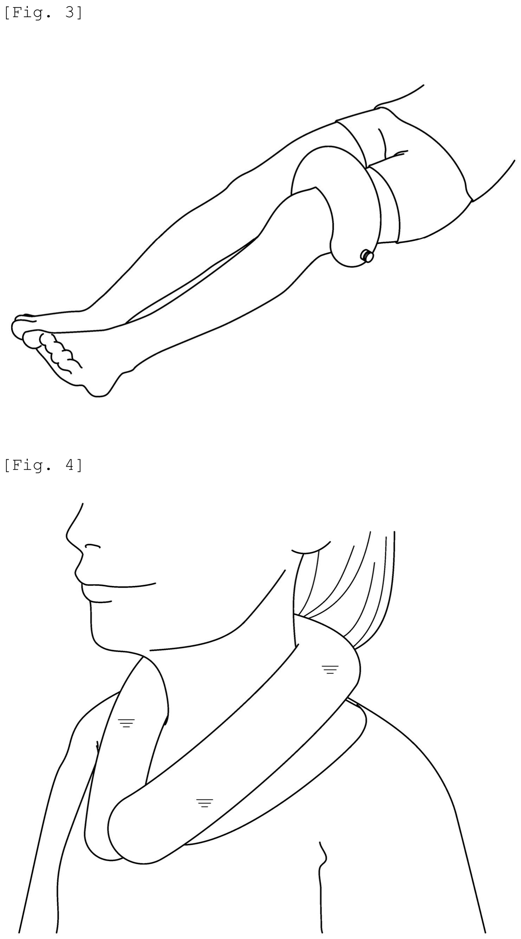

An overall configuration of a fluid sealed container 100 according to the present embodiment will be described with reference to Figs. 1 to 4. Fig. 1 is a diagram showing an example of the fluid sealed container 100 according to the present embodiment before carbon dioxide gas-containing cold water or the like is injected into the fluid sealed container 100, and Fig. 2 is a diagram showing a cooling tool obtained by enclosing carbon dioxide gas-containing cold water in a modified example of the fluid sealed container 100 according to the present embodiment. Fig. 3 is a schematic diagram showing an example in which a cooling tool obtained by enclosing carbon dioxide gas-containing cold water in the fluid sealed container 100 according to the present embodiment is used by being worn around a leg. Fig. 4 is a schematic diagram showing an example in which a cooling tool obtained by enclosing carbon dioxide gas-containing cold water in the modified example of the fluid sealed container 100 according to the present embodiment is used by being worn around a neck.

The fluid sealed container 100 according to the present embodiment is a fluid sealed container in which carbon dioxide gas-containing cold water of equal to or less than 15 degrees centigrade is enclosed to be usable as a human body cooling tool, for example, as shown in Figs. 1 and 2, the fluid sealed container 100 including a main body part 31 formed of a film made of a thermoplastic elastomer or a thermoplastic resin, and an injection port part 21 that is connected to the main body part 31 and is configured to be openable and closable to be able to hermetically seal the main body part 31. The carbon dioxide gas-containing cold water or the like is injected into and sealed in the main body part 31 from the injection port part 21, and if necessary, is further cooled, making it possible to provide a cooling tool used as shown in Fig. 3 or 4, for example. Furthermore, the cooling tool can be used by replacing the carbon dioxide gas-containing cold water or the like enclosed in the cooling tool from the injection port part 21, that is, the cooling tool can be repeatedly used.

In the embodiment in Fig. 2, the main body part 31 includes two branch portions 31-1 formed toward both sides by bifurcating from the injection port part 21, and is curved or bent (for example, an embodiment in which a curvature radius of the curved inner periphery is equal to or less than 15 cm). This makes the cooling tool more suitable for use by being worn around and stably in close contact with a user's neck, leg or the like.

<Main Body Part>

First of all, a configuration of the main body part 31 will be described. A configuration of a film forming the main body part 31 will be described later.

The main body part 31 is a member having a bag shape or the like, the member having an internal space in which carbon dioxide gas-containing cold water of equal to or less than 15 degrees centigrade can be enclosed, that is, a member serving as a main body of a cooling tool or the like. The main body part 31 may have any configuration that enables at least carbon dioxide gas-containing cold water of equal to or less than 15 degrees centigrade to be enclosed therein, and, for example, may have a configuration that enables a solid such as dry ice and gas to be enclosed therein. Alternatively, the main body part 31 may have a configuration that enables a liquid (carbon dioxide gas-containing warm water or the like) other than cold water to be enclosed therein. Here, the term "enclose" means that a liquid or the like is housed and sealed in the internal space of the main body part 31, but in this case, a certain amount of carbon dioxide gas may be able to penetrate the film forming the main body part 31 as described later.

The main body part 31 preferably has a configuration in which at least one cross section of the internal space has a circular shape or an elliptical shape in a state in which the carbon dioxide gas-containing cold water of equal to or less than 15 degrees centigrade is enclosed, and in particular, more preferably has a configuration in which at least a portion of the cross section of the internal space in a lateral direction has a circular shape or an elliptical shape. This is because the main body part 31 is easily brought into contact with and adheres to each portion of the human body. Here, the term "circular shape" means a substantially circular shape, and the present invention encompasses embodiments wherein the above-described shape of the cross section has not only a perfect circular shape but also a circular shape in which a value obtained by dividing a long diameter by a short diameter is equal to or less than 2, and further equal to or less than 1.5. The term "elliptical shape" means a substantially elliptical shape, in which a value obtained by dividing a long diameter by a short diameter is more than 2 and equal to or less than 10.

However, the shape of the main body part 31 is not limited to the above-described shapes, and may be any other shape, such as a pillow shape and an ice pack shape, that can be used by contacting at least a portion of the human body and has an internal space in which the carbon dioxide gas-containing cold water of equal to or less than 15 degrees centigrade can be enclosed.

In addition, the size of the main body part 31 is not limited to particular size, but since the main body part 31 when used as the cooling tool is easily brought into contact with each portion of the human body, the volume of the internal space (a region in which the carbon dioxide gas-containing cold water of equal to or less than 15 degrees centigrade is capable of being enclosed) is preferably equal to or more than 50 cm³ (0.05 L) and equal to or less than 2000 cm³ (2.0 L) per one container. The lower limit may be equal to or more than 100 cm³ (0.1 L) or may be equal to or more than 120 cm³ (0.12 L). The upper limit may be equal to or less than 1500 cm³ (1.5 L) or may be equal to or less than 1200 cm³ (1.2 L).

### <Injection Port Part>

Next, a configuration of the injection port part 21 connected to the above-described main body part 31 will be described.

The injection port part 21 is connected to the main body part 31, and has a configuration in which the carbon dioxide gas-containing cold water or the like can be injected into and sealed in the main body part 31 therefrom and the enclosed carbon dioxide gas-containing cold water or the like can be discharged therefrom. That is, the injection port part 21 is configured to be openable and closable, and is configured to be capable of hermetically sealing the main body part 31. Accordingly, the injection port part 21 may have any configuration as long as it has such a configuration, but as a preferred example, the injection port part 21 includes a detachable cap and a cap attachment portion as shown in Figs. 1 to 4, for example, in which a male thread shape and a female thread shape (a spiral thread portion) are formed on an inner periphery of the cap and an outer periphery of the cap attachment portion, and the cap and the cap attachment portion threadedly engage with each other so as to be detachable from and attachable to each other and to enable hermetic sealing. Furthermore, the cap may be configured to be able to be completely detached from the fluid sealed container 100 or may be configured to be coupled with the cap attachment portion by a hinge or the like. Alternatively, the injection port part 21 may have a non-detachable configuration such as a type (slide type) of opening and closing an opening by sliding a cap-like member, and further may have a pinching type (hermetically sealing type by a pinch made of resin, metal or the like) configuration.

The fluid sealed container 100 according to the present embodiment that includes such an injection port part 21 and the main body part 31 formed of a film as described later not only can enclose the carbon dioxide gas-containing cold water or the like in the main body part 31 to serve as the cooling tool but also can be repeatedly used as the cooling tool by replacing the carbon dioxide gas-containing cold water or the like inside the main body part 31 from the injection port part 21.

In the fluid sealed container 100 according to the present embodiment, one or more injection port parts 21 as described above are connected to any region of the main body part 31 so as to communicate with the internal space. The connection region for the injection port part 21 is not limited to a particular connection region, but, when the fluid sealed container 100 serves as the cooling tool, the injection port part 21 is preferably connected to a region hard to contact the human body.

The size of the injection port part 21 is not limited, and, for example, an opening diameter of the injection port part 21 has a size sufficient to easily inject the carbon dioxide gas-containing cold water or the like. Furthermore, the injection port part 21 preferably has the size and material capable of functioning also as a gripping part when the carbon dioxide gas-containing cold water or the like is injected into the fluid sealed container 100 according to the present embodiment or when the obtained cooling tool is used. Preferred examples of the material of such an injection port part 21 include resin and metal, but from the standpoint of weight reduction or the like of the fluid sealed container 100 according to the present embodiment, the injection port part 21 is more preferably made of resin material such as polyethylene (PE), polypropylene (PP), polyvinyl chloride (PVC), polystyrene (PS), nylon (Ny), acrylonitrile-butadiene-styrene (ABS) copolymer, polyethylene terephthalate (PET), polylactic acid (PLA), polycarbonate (PC), an epoxy resin, or an urethane resin. This also applies to the case where the above-described cap is provided.

The carbon dioxide gas-containing cold water or the like is enclosed in the fluid sealed container 100 according to the present embodiment that includes the main body part 31 and the injection port part 21 as described above to serve as the cooling tool, and as shown in Fig. 3 or 4, it is very preferable that the cooling tool is used so that the main body part 31 contacts each portion of the human body (for example, directly contacts the skin of each portion of the human body or contacts each portion of the human body through cloth or clothing which the carbon dioxide gas can penetrate).

It is also preferable that a plurality of cooling tools configured as described above are used and are arranged at predetermined positions to be used as wearing tools (wearing cooling tools). The plurality of cooling tools may be configured so that these internal spaces communicate with one another via tubes or the like, making it possible to move the enclosed carbon dioxide gas-containing cold water among the plurality of cooling tools. Also in the embodiment of the wearing tool, it is very preferable that the cooling tool is used to contact each portion of the human body (for example, directly contacts the skin).

### (Film Forming Main Body Part)

Next, the film made of a thermoplastic elastomer or a thermoplastic resin, the film forming the main body part 31 of the fluid sealed container 100 according to the present embodiment, will be described in detail.

The film forming the main body part 31 of the fluid sealed container 100 according to the present embodiment is made of a thermoplastic elastomer or a thermoplastic resin as described above, is water-impermeable, has a glass transition temperature of less than 0 degrees centigrade, and has a carbon dioxide gas penetration of equal to or more than 5 L/(m²·24 hr·atm) as measured under the condition of 10 degrees centigrade in accordance with JIS K 7126-2 Annex B, and further has a tensile strength of equal to or more than 8 N/10 mm as measured under the condition of 10 degrees centigrade in accordance with JIS K 7161-1. Hereinafter, these will be described in detail.

### <Thermoplastic Elastomer or Thermoplastic Resin>

The film forming the main body part 31 of the fluid sealed container 100 according to the present embodiment is made of a thermoplastic elastomer or a thermoplastic resin. That is, this is a film containing a thermoplastic elastomer or a thermoplastic resin as a main component. The terms "containing as a main component" means containing equal to or more than 80% as a percentage by mass, more preferably, containing equal to or more than 90%, and further preferably, containing equal to or more than 95%, or the film may be made of 100% thermoplastic elastomer or thermoplastic resin.

Here, the "thermoplastic elastomer (TPE)" is a material having both of the thermoplastic properties and the elastomer properties (elastic properties), may be any thermoplastic elastomer that can form the above-described film, and is preferably, for example, thermoplastic polyurethane elastomer (thermoplastic polyurethane elastomer, TPU), thermoplastic polyolefin elastomer (thermoplastic olefinic elastomer, TPO), thermoplastic polystyrene elastomer (thermoplastic styrenic elastomer, TPS), or the like. The above-described film is preferably made of one or more selected from the group consisting of those mentioned above.

A thermoplastic resin may be any material having a thickness that is capable of forming the film satisfying the above-described water impermeability, glass transition temperature, carbon dioxide gas penetration, and tensile strength, but the film is preferably made of polyolefin such as polyethylene, and in particular, the film is more preferably made of linear low-density polyethylene (LLDPE).

The above-described film is more preferably made of a thermoplastic polyurethane elastomer or a thermoplastic polyolefin elastomer from the standpoint of feel or the like when directly contacting the skin. In particular, also in light of the effects of easily and highly achieving both of the followability to the human body at the low temperature and the durability at the low temperature while maintaining the carbon dioxide gas penetration at the low temperature, the film is further preferably made of a thermoplastic polyurethane elastomer. The thermoplastic polyurethane elastomer may be ether-based or ester-based, but the film made of an ether-based thermoplastic polyurethane elastomer can maintain the high carbon dioxide gas penetration at the low temperature while highly achieving both of the followability to the human body at the low temperature and the durability at the low temperature, and, in addition, can show excellent water resistance. Furthermore, the film made of an ether-based thermoplastic polyurethane elastomer also shows excellent wear resistance.

Here, the "ether-based thermoplastic polyurethane elastomer" has an ether bond (-O-) in the main chain of a thermoplastic polyurethane elastomer molecule, and the "ester-based thermoplastic polyurethane elastomer" has an ester bond (-COO-) in the main chain of a thermoplastic polyurethane elastomer molecule.

In a case where the above-described film is made of the thermoplastic polyurethane elastomer, the film more preferably has the rubber hardness of equal to or more than 50 and equal to or less than 85 as measured in accordance with JIS K 6253 using a durometer of type A (for example, a digital durometer EA760AS-11 manufactured by IKEDA SCIENTIFIC Co., Ltd. or the like). This is because it is easy to also maintain the high carbon dioxide gas penetration at the low temperature while highly achieving both of the followability to the human body at the low temperature and the durability at the low temperature. From the standpoint of the carbon dioxide penetration or the like at the low temperature, the upper limit is further preferably equal to or less than 83, and still more preferably equal to or less than 80. From the standpoint of the durability or the like, the lower limit is further preferably equal to or more than 55, still more preferably equal to or more than 60, still further preferably equal to or more than 65, yet more preferably more than 70, and yet still more preferably equal to or more than 75. The film made of the ether-based thermoplastic polyurethane elastomer is preferable because it tends to have the rubber hardness within the above-described range.

### <Water Impermeability>

The film forming the main body part 31 of the fluid sealed container 100 according to the present embodiment is water-impermeable as a whole. This makes it possible to, when the carbon dioxide gas-containing cold water is enclosed in the fluid sealed container 100 according to the present embodiment to serve as the cooling tool, substantially eliminate loss of the enclosed cold water during the use or the like. As used herein, the water impermeability means that a substance in a liquid state, such as water cannot penetrate the film. However, the film may have the penetration of water vapor which is gas in addition to the carbon dioxide gas penetration, which will be described later. For example, the water vapor penetration of the film may be equal to or more than 5 g/ (m²·24 hr) and equal to or less than 200 g/ (m²·24 hr) as measured under the condition of 25 degrees centigrade, 90% RH in accordance with JIS K 7129.

### <Glass Transition Temperature>

The film forming the main body part 31 of the fluid sealed container 100 according to the present embodiment has the glass transition temperature (Tg) of less than 0 degrees centigrade. This makes it difficult to, when the carbon dioxide gas-containing cold water of equal to or less than 15 degrees centigrade is enclosed to be used as the human body cooling tool, easily lower the fit feeling and generate breakage or crack due to excessive hardening of the main body part 31. The glass transition temperature is more preferably equal to or less than -5 degrees centigrade, further preferably equal to or less than -10 degrees centigrade, still more preferably equal to or less than -15 degrees centigrade, and still further preferably equal to or less than -18 degrees centigrade. From the standpoint of the durability or the like, the lower limit is further preferably equal to or more than - 120 degrees centigrade, still more preferably equal to or more than -90 degrees centigrade, still further preferably equal to or more than -60 degrees centigrade, and yet more preferably equal to or more than -40 degrees centigrade.

### <Carbon Dioxide Gas Penetration>

The film forming the main body part 31 of the fluid sealed container 100 according to the present embodiment has a carbon dioxide gas penetration of equal to or more than 5 L/(m²·24 hr·atm)) as measured under the condition of 10 degrees centigrade in accordance with JIS K 7126-2 Annex B. That is, the carbon dioxide gas penetration of the film is equal to or more than 5 L/(m²·24 hr·atm) as measured by a predetermined apparatus under an environment of 10 degrees centigrade in accordance with JIS K 7126-2 (gas penetration measurement method, equal pressure method) Annex B (test method by gas chromatography). This enables, when the carbon dioxide gas-containing cold water is enclosed in the fluid sealed container 100 according to the present embodiment to serve as the cooling tool, the carbon dioxide gas to sufficiently permeate the skin or the like followed and contacted by the film forming the main body part 31 so that the blood circulation of the cooling portion can be maintained or promoted. This makes it possible to obtain the alleviation effect on fatigue or swelling after exercise or the like and easily extend the cooling effect not locally but throughout the body.

From the standpoint of achieving both of the carbon dioxide gas penetration and the durability, the carbon dioxide gas penetration is more preferably equal to or more than 7 L/(m²·24 hr·atm), further preferably equal to or more than 10 L/(m²·24 hr·atm)), still more preferably equal to or more than 12 L/(m²·24 hr·atm), still further preferably equal to or more than 15 L/(m²·24 hr·atm), yet more preferably equal to or more than 18 L/(m²·24 hr·atm), and yet further preferably equal to or more than 20 L/(m²·24 hr·atm). The upper limit is preferably equal to or less than 100 L/(m²·24 hr.atm), more preferably equal to or less than 80 L/(m²·24 hr·atm), and further preferably equal to or less than 50 L/(m²·24 hr·atm). This makes it possible to, when the fluid sealed container 100 serves as the cooling tool, sufficiently transfer the carbon dioxide gas from the carbon dioxide gas-containing cold water or the like to the skin or the like through the film, so that the blood circulation can be maintained or promoted, and the durability can also be easily obtained.

The carbon dioxide gas penetration can be adjusted by the constituent material of the film forming the main body part 31 of the fluid sealed container 100 according to the present embodiment, and the total thickness of the constituent material.

### <Tensile Strength at 10 Degrees Centigrade>

The film forming the main body part 31 of the fluid sealed container 100 according to the present embodiment has a tensile strength of equal to or more than 8 N/10 mm as measured under the condition of 10 degrees centigrade in accordance with JIS K 7161-1. Thus, the main body part 31 of the fluid sealed container 100 according to the present embodiment shows excellent durability at the low temperature. The lower limit is more preferably equal to or more than 10 N/10 mm, further preferably equal to or more than 15 N/10 mm, still more preferably equal to or more than 20 N/10 mm, still further preferably equal to or more than 30 N/10 mm, and yet more preferably equal to or more than 40 N/10 mm. The upper limit may be equal to or less than 150 N/10 mm, may be equal to or less than 130 N/10 mm, may be equal to or less than 100 N/10 mm, may be equal to or less than 80 N/10 mm, or may be equal to or less than 60 N/10 mm.

As used herein, the "tensile strength as measured under the condition of 10 degrees centigrade in accordance with JIS K 7161-1" means a physical property reflecting the countermeasure for cracks (high-speed and local deformation) during use at the low temperature as measured on the basis of JIS K 7161-1 (Plastics - Determination of tensile properties), in which a test piece cut in a rectangular shape of 10 mm in width × 70 mm in length from the film is prepared and is stored under an environment of 10 degrees centigrade for 24 hours, while Tensilon UTC-100W manufactured by ORIENTEC CO., LTD. is prepared for the measurement conditions of an inter-chuck distance (an attachment interval distance of the test piece in a state in which the test piece before tension is attached) of 30 mm, a head speed of 300 mm/minute (tensile speed), and the normal test mode under an environment of 23 degrees centigrade, and the test piece is taken out from the environment of 10 degrees centigrade for each measurement, the tensile test is performed under the above-described conditions using the above-described Tensilon UTC-100W manufactured by ORIENTEC CO., LTD. within 2 minutes, the breaking strength (tensile strength when the film has broken, N/10 mm) is measured, and an average value obtained from three measurements is determined as the tensile strength.

The tensile strength can also be adjusted by the constituent material of the film forming the main body part 31 of the fluid sealed container 100 according to the present embodiment, and the total thickness of the constituent material.

### <Percentage Elongation When Load of 0.98 N is Applied at 10 Degrees Centigrade>

The film forming the main body part 31 of the fluid sealed container 100 according to the present embodiment is not limited, but tends to have more excellent followability to the human body at the low temperature, and therefore, is as described above and more preferably has the percentage elongation of equal to or more than 2% when the load of 0.98 N (100 g weight) is applied under the condition of 10 degrees centigrade in accordance with JIS K 7161-1. That is, the ductility at 10 degrees centigrade is more preferably equal to or more than a certain value. The lower limit is further preferably equal to or more than 3%, and still more preferably equal to or more than 4%. From the standpoint of the durability or the like, the upper limit is more preferably equal to or less than 20%, further preferably equal to or less than 18%, still further preferably equal to or less than 15%.

As used herein, the "percentage elongation when a load of 0.98 N is applied as measured under the condition of 10 degrees centigrade in accordance with JIS K 7161-1" means a physical property reflecting the countermeasure (initial response to an impact force) for cracks during use at the low temperature as measured on the basis of JIS K 7161-1 (Plastics - Determination of tensile properties), in which a test piece cut in a rectangular shape of 10 mm in width × 150 mm in length from the film is prepared and is stored under an environment of 10 degrees centigrade for 24 hours, while Tensilon UTC-100W manufactured by ORIENTEC CO., LTD. is prepared for the measurement conditions of an inter-chuck distance of 100 mm, a head speed of 300 mm/minute, and the cycle test mode under an environment of 23 degrees centigrade, and the test piece is taken out from the environment of 10 degrees centigrade for each measurement, the percentage elongation (a ratio of an elongated length when the film length before elongation is defined as 100%) when a load of 0.98 N is applied in a direction of elongation (elongating direction) under the above-described conditions using the above-described Tensilon UTC-100W manufactured by ORIENTEC CO., LTD. within 2 minutes, and an average value obtained from three measurements is determined as the percentage elongation. That is, it can be said that this represents the ease of elongation of the film at the low temperature.

The percentage elongation can also be adjusted by the constituent material of the film forming the main body part 31 of the fluid sealed container 100 according to the present embodiment, and the total thickness of the constituent material.

### <Permanent Strain after 100% Elongation at 10 Degrees Centigrade>

The film forming the main body part 31 of the fluid sealed container 100 according to the present embodiment is not limited, but tends to have more excellent followability to the human body at the low temperature, and durability at the low temperature, and therefore, is as described above and more preferably has the permanent strain of equal to or less than 20% after 100% elongation as measured under the condition of 10 degrees centigrade in accordance with JIS K 7161-1. The upper limit is further preferably equal to or less than 15%, still more preferably equal to or less than 13%, and still further preferably equal to or less than 10%. The film made of the ether-based thermoplastic polyurethane elastomer is preferable because it tends to have the permanent strain within the above-described range.

As used herein, the "permanent strain after 100% elongation as measured under the condition of 10 degrees centigrade in accordance with JIS K 7161-1" means a physical property reflecting the countermeasure (recovery against strain) for repeated deformation during use at the low temperature as measured on the basis of JIS K 7161-1 (Plastics - Determination of tensile properties), in which a test piece cut in a rectangular shape of 10 mm in width × 150 mm in length from the film is prepared and is stored under an environment of 10 degrees centigrade for 24 hours, while Tensilon UTC-100W manufactured by ORIENTEC CO., LTD. is prepared for the measurement conditions of an inter-chuck distance of 100 mm, a head speed of 300 mm/minute, and the cycle test mode under an environment of 23 degrees centigrade, and the test piece is taken out from the environment of 10 degrees centigrade for each measurement, the 100% elongation is performed under the above-described conditions using the above-described Tensilon UTC-100W manufactured by ORIENTEC CO., LTD. within 2 minutes, and then, the head is returned up to 0% at the same head speed, the percentage elongation (a ratio of an elongated length when the film length before elongation is defined as 100%) when the elongation stress becomes 0 N in the recovery direction (returning direction) is measured, and an average value obtained from three measurements is determined as the percentage elongation. That is, it can be said that this represents the ease of returning to the original length when the film is elongated at the low temperature. The permanent strain can also be adjusted by the constituent material of the film forming the main body part 31 of the fluid sealed container 100 according to the present embodiment, and the total thickness of the constituent material.

The above-described percentage elongation and permanent strain are measured using the above-described device (Tensilon UTC-100W manufactured by ORIENTEC CO., LTD. or the like) in the elongating direction and the returning direction in the cycle test set as described above, so that the respective numerical values can be read from the obtained measurement results.

Here, the characteristics in which a predetermined percentage elongation is large at the low temperature and a predetermined permanent strain is small at the low temperature are more preferable in that the cooling tool is less likely to break when being dropped down during use at the low temperature and the followability to the human body can be achieved. That is, to prevent the cooling tool from receiving damage such as cracks and breakage when an impact force is applied to the cooling tool, any of the characteristics of (1) the film has the strength that can endure the damaging force (for example, a non-elastomer thick film such as an LLDPE film of 100 µm) and (2) the film is elastic to be able to follow the deformation instantaneously in response to rapid deformation stress is necessary, but in a case of (1), the followability to the human body is insufficient because of poor deformability in spite of robustness. The film described in (1) causes and accumulates the permanent strain in a case where a load partially concentrates due to repeated use, which may easily lead to damage. On the other hand, as described above, when a predetermined percentage elongation is large at the low temperature or a predetermined permanent strain is small at the low temperature, the possible risk can be extremely reduced and the high followability to the human body is easily obtained.

### <Melting Point>

The film forming the main body part 31 of the fluid sealed container 100 according to the present embodiment is not limited, but tends to have more excellent heat sealability, and therefore, the melting point (Tm) is more preferably equal to or more than 120 degrees centigrade and equal to or less than 150 degrees centigrade. The lower limit is further preferably equal to or more than 125 degrees centigrade, and still more preferably more than 130 degrees centigrade. The upper limit is further preferably equal to or less than 145 degrees centigrade, and still more preferably equal to or less than 140 degrees centigrade.

Furthermore, in a case where the main body part 31 of the fluid sealed container 100 according to the present embodiment is formed by the above-described film using a heat seal, the seal strength is more preferably equal to or more than 10 N/10 mm, and further preferably equal to or more than 15 N/10 mm.

As used herein, the "seal strength" means that a test piece of 10 mm in width × 70 mm in length including a seal portion (1 mm in width of the seal portion, that is, a seal portion of 10 mm × 1 mm) is cut from the film forming the main body part 31, and the seal strength is measured under the measurement conditions of an inter-chuck distance of 30 mm, a head speed of 300 mm/minute, and the normal test mode using Tensilon UTC-100W manufactured by ORIENTEC CO., LTD. on the basis of the method of JIS K 6854-3 (T-peel test) except that the test piece has a different size, and an average value obtained from three measurements is determined as the seal strength.

### <Total Thickness of Film>

In the film forming the main body part 31 of the fluid sealed container 100 according to the present embodiment, the total thickness of the film may be appropriately set according to the constituent material or the like so as to be able to maintain the above-described physical properties, but the total thickness is preferably equal to or more than 50 µm and less than 250 µm because all of the followability to the human body at the low temperature, the durability at the low temperature, and the carbon dioxide gas penetration at the low temperature can be easily achieved. The lower limit is further preferably equal to or more than 80 µm, still more preferably equal to or more than 100 µm, and still further preferably equal to or more than 120 µm. The upper limit is further preferably equal to or less than 230 µm, and still more preferably equal to or less than 200 µm.

Here, the "total thickness" of the film is a full length in the thickness direction of the film forming the main body part, that is, a total thickness of all layers forming the film. Accordingly, in the film in which a plurality of layers including a coating layer are laminated, the thicknesses of all the layers are summed up.

Such a fluid sealed container 100 according to the present embodiment can be manufactured by a method of joining and forming the above-described film including a single layer or a plurality of laminated layers by a heat seal to form the main body part 31 having the above-described shape, and further connecting the above-described injection port part 21 at a predetermined position of the main body part 31.

The fluid sealed container 100 according to the present embodiment as described above can be used as a human body cooling tool by enclosing carbon dioxide gas-containing cold water of equal to or less than 15 degrees centigrade, and has the carbon dioxide gas penetration of equal to or more than a predetermined value at the low temperature, and is excellent in both of the followability to the human body at the low temperature and the durability at the low temperature. Accordingly, the cooling tool obtained by enclosing the carbon dioxide gas-containing cold water of equal to or less than 15 degrees centigrade in the fluid sealed container 100 is applied to be worn around a portion of the user's body, in particular, a leg, a neck or the like to cause the cooling tool to directly contact the skin (for example, by cooling by applying the cooling tool as shown in Fig. 3 or 4), making it possible to sufficiently exert the cooling function of the cold water from the fit feeling and the carbon dioxide gas penetration and the function by the carbon dioxide gas permeation, and obtain the high cooling action, the action of maintaining of promoting blood circulation, and the like. The cooling effect is easily obtained not only in a portion that the cooling tool contacts but also in a wide range of the human body. Furthermore, the cooling tool is unlikely to generate breakage or cracks during use or the like.

For example, it is preferable that the carbon dioxide gas-containing cold water having a temperature of equal to or less than 15 degrees centigrade and a carbon dioxide gas concentration of equal to or more than 300 ppm is enclosed in the fluid sealed container 100 according to the present embodiment to serve as the cooling tool. The concentration of the carbon dioxide gas in the cold water is not limited, but is more preferably equal to or more than 400 ppm, still more preferably equal to or more than 500 ppm, and still further preferably equal to or more than 700 ppm. The upper limit may be equal to or less than 1500 ppm, may be equal to or less than 1200 ppm, or may be equal to or less than 1000 ppm. Note that the carbon dioxide gas concentration refers to a concentration obtained assuming that in the range above a saturation amount of the carbon dioxide gas in the cold water, the carbon dioxide gas is dissolved in the cold water. The water temperature may be any water temperature that is equal to or less than 15 degrees centigrade (equal to or more than 0 degrees centigrade and equal to or less than 15 degrees centigrade), but may be, for example, equal to or less than 10 degrees centigrade.

As described above, the carbon dioxide gas-containing cold water of equal to or less than 15 degrees centigrade is enclosed in the fluid sealed container 100 according to the present embodiment to be usable as the cooling tool, but the present invention encompasses embodiments wherein not only the carbon dioxide gas-containing cold water of equal to or less than 15 degrees centigrade is filled and sealed to serve as the cooling tool, but also the carbon dioxide gas-containing water is filled and sealed and then, the entire container is cooled by cold water of equal to or less than 15 degrees centigrade, ice or the like to serve as the cooling tool. Accordingly, in a case where the temperature of the enclosed carbon dioxide gas-containing cold water rises during use as the cooling tool, cooling the cooling tool as it is by cold water, ice or the like makes it possible to decrease the temperature of the enclosed carbon dioxide gas-containing water. Together with the cold water or the like, a carbon dioxide gas generating agent (for example, a solid, powder, or granular carbon dioxide gas generating agent) that generates carbon dioxide gas when being mixed with and dissolved in the cold water or the like may be enclosed and stirred in the fluid sealed container 100 according to the present embodiment. This fluid sealed container 100 may be further cooled as described above.

Furthermore, the fluid sealed container 100 according to the present embodiment can be used not only as the cooling tool by enclosing the carbon dioxide gas-containing cold water of equal to or less than 15 degrees centigrade, but also in other applications. For example, carbon dioxide gas-containing warm water may be enclosed in the fluid sealed container 100 to be usable as a warming tool. When the warming tool is used by being worn around a leg, a neck or the like, the hot heat and carbon dioxide gas permeation make it possible to obtain the high warming action and the action of promoting blood circulation. Also in this case, the carbon dioxide gas concentration of the carbon dioxide gas-containing warm water is preferably equal to or more than 300 ppm as in the case of the above-described carbon dioxide gas-containing cold water.

A description will be given below of Examples and the like in the present invention, but the present invention is not limited to the Examples and the like described below, and various modifications are possible within the technical concept of the present invention.

### Examples

In a film or sheet made of a material shown in Table 1 described below, a main body part having a shape shown in Fig. 2 was formed using a heat seal, and a fluid sealed container for each of Examples 1 to 8 and Comparative Examples 1 to 3 was prepared. Then, 400 g of carbon dioxide gas-containing cold water having a temperature of 10 degrees centigrade and a carbon dioxide gas concentration of 1000 ppm was enclosed in the fluid sealed container, and each cooling tool was prepared.

Details of each film or sheet are as described below. Each film or sheet is water-impermeable.
· Examples 1 to 3: A film made of an ether-based thermoplastic polyurethane elastomer (Elastollan 1180A, manufactured by BASF)
· Examples 4 to 6: A film made of an ester-based thermoplastic polyurethane elastomer (Elastollan C85A, manufactured by BASF)
· Example 7: A film made of an ester-based thermoplastic polyurethane elastomer (Elastollan C90A, manufactured by BASF)
· Example 8: A film made of a linear low-density polyethylene (LLDPE) (manufactured by SEISANNIPPONSHA LTD)
· Comparative Example 1: A film made of a linear low-density polyethylene (LLDPE) (manufactured by TRUSCO NAKAYAMA CORPORATION)
· Comparative Example 2: A film made of 4-methyl-1-pentene- α olefin copolymer (ABSORTOMER manufactured by Mitsui Chemicals, Inc.)
· Comparative Example 3: A sheet having a three-layer structure including films as outer layers made of 4-methyl-1-pentene-α olefin copolymer (ABSORTOMER manufactured by Mitsui Chemicals, Inc.) and a film as an intermediate layer made of a styrene-based elastomer (SEBS, SEPTON 2063, manufactured by Kuraray) which is a block copolymer of polystyrene, polyethylene, and polybutylene.

Regarding each film or sheet forming the main body part, the carbon dioxide gas penetration (L/(m²·24 hr·atm)) at 10 degrees centigrade, the percentage elongation (%) when a load of 0.98 N was applied at 10 degrees centigrade, the permanent strain (%) after 100% elongation at 10 degrees centigrade, the tensile strength (N/10 mm) at 10 degrees centigrade, and the seal strength (N/10 mm) were confirmed as follows in advance.

### <Carbon Dioxide Gas Penetration at 10 Degrees Centigrade>

The carbon dioxide gas penetration of each film or sheet was measured under a measurement environment at 10 degrees centigrade in accordance with JIS K 7126-2 (gas penetration measurement method, equal pressure method) Annex B (test method by gas chromatography). For measurement, a gas penetration measuring device (GTR-10XFKS, gas chromatography) manufactured by GTR Tec Corporation was used.

### <Percentage Elongation When Load of 0.98 N is Applied at 10 Degrees Centigrade>

A plurality of test pieces cut in a rectangular shape of 10 mm in width × 150 mm in length from each film or sheet were prepared and were stored under an environment of 10 degrees centigrade for 24 hours on the basis of JIS K 7161-1 (Plastics - Determination of tensile properties). Furthermore, Tensilon UTC-100W manufactured by ORIENTEC CO., LTD. was prepared for the following measurement conditions under an environment of 23 degrees centigrade.
· Inter-chuck distance: 100 mm
· Head speed: 300 mm/minute
· Cycle test mode
Then, the following numerical value was measured.
· Percentage elongation when a load of 0.98 N is applied in an elongation direction (elongating direction)

The test piece was taken out from the environment of 10 degrees centigrade for each measurement, and the measurement was made within 2 minutes. The measurement result was obtained as an average value of N = 3.

### <Permanent Strain after 100% Elongation at 10 Degrees Centigrade>

In the above-described percentage elongation test based on JIS K 7161-1 (Plastics - Determination of tensile properties -), the test pieces were prepared for the above-described conditions (an inter-chuck distance of 100 mm, a head speed of 300 mm/minute, and the cycle test mode) and 100% elongation was performed, and the head was returned up to 0% (head speed: 300 mm/minute), and the percentage elongation when the elongation stress became 0 N in the recovery direction (returning direction) was measured as the permanent strain after 100% elongation at 10 degrees centigrade. The measurement result was obtained as an average value of N = 3.

### <Tensile Strength at 10 Degrees Centigrade>

On the basis of JIS K 7161-1 (Plastics - Determination of tensile properties -), the test pieces cut in a rectangular shape of 10 mm in width × 70 mm in length were prepared in the same manner as the above-described percentage elongation measurement, the tensile test was performed under the following conditions using Tensilon UTC-100W manufactured by ORIENTEC CO., LTD., and the breaking strength (tensile strength when the test piece was broken) was measured.
· Inter-chuck distance: 30 mm
· Head speed: 300 mm/minute
· Normal test mode
The measurement result was obtained as an average value of N = 3.

### <Seal Strength>

The test pieces of 10 mm in width × 70 mm in length including a seal portion were cut, and the seal strength was measured under the following measurement conditions on the basis of the method described in JIS K 6854-3 except that the test piece has a different size (T-peel test).
· Device: Tensilon UTC-100W manufactured by ORIENTEC CO., LTD.
· Test piece width: 10 mm
· Length in seal test direction: 1 mm (seal width) · Inter-chuck distance: 30 mm
· Head speed: 300 mm/minute
The measurement result was obtained as an average value of N = 3.

These results are shown in Table 1 below. And, the glass transition temperature (Tg: degrees centigrade), the melting point (Tm: degrees centigrade), the rubber hardness measured in accordance with JIS K 6253 using type A of durometer (digital durometer EA760AS-11, manufactured by IKEDA SCIENTIFIC Co., Ltd.), and the total thickness (µm) of each film or sheet are shown in Table 1 below.

The skin flushing, the coolness, the cooling sensation on the head, the fit feeling, and the general use feeling when each prepared cooling tool was brought into contact with the vicinity of the neck of the user were evaluated as follows. The skin flushing, the cool feeling, and the cooling sensation on the head are evaluated mainly for the carbon dioxide gas penetration at the low temperature and the followability at the low temperature, and when a sufficient amount of carbon dioxide gas reaches the skin surface, the skin flushing is generated by the action of promoting blood circulation and the cooling effect spreads to the head or the like.

### <Skin Flushing>

After the cooling tool was worn around the neck of one monitor for five minutes, the skin of the contact portion was visually observed, and if redness on the skin was observed, the result was indicated by O, and if no, the result was indicated by ×.

### <Coolness>

Three expert panelists each wore the cooling tool around the neck to directly contact the skin and used it for five minutes, and the evaluation was made as follows. The evaluation environment was 30 degrees centigrade, 60% RH, an environmental adaptation was performed for 30 minutes in advance, and the evaluation was made (N = 3).

As the coolness after use for five minutes, the level closest to the evaluation result among the following three-level evaluation was selected, and an average value of the three panelists was calculated. The average value was rounded to an integer.
3 The coolness comfortably spreads without concentrating at the neck or is actually felt in a wide range.
2 The coolness is felt on the neck with an unpleasant feeling. Actual cool feeling slightly spreads not just on the neck.
1 The coolness is felt on only the contact portion with the neck and is painful.

### <Cooling Sensation on Head>

The following evaluation was made in the same manner as the above-described coolness. As the cooling sensation on the head after use for five minutes, the level closest to the evaluation result among the following three-level evaluation was selected, and an average value of the three panelists was calculated. The average value was rounded to an integer.
3 The cooling sensation on the head is felt immediately after the cooling tool is worn, and the head is cold after use for five minutes.
2 The cooling sensation on the head is felt after use for five minutes.
1 The cooling sensation on the head is not felt.

### <Fit Feeling>

The following evaluation was made in the same manner as the above-described coolness.

During use for five minutes, the softness of the worn cooling tool, the fit feeling, and the feeling of adhering to the neck were evaluated, the level closest to the evaluation result among the following three-level evaluation was selected, and an average value of the three panelists was calculated. The average value was rounded to an integer.
3 The cooling tool is soft even immediately after being worn, and fits the curved surface of the neck. The cooling tool adheres to the skin. The cooling tool fits the wearer continuously while being worn.
2 The cooling tool is slightly stiff and is not felt to adhere to the skin, but tentatively fits the curved surface of the neck. Alternatively, the cooling tool tentatively fits the wearer and is felt bad due to stickiness.
1 The cooling tool is hard, and does not fit the neck.

### <General Use Feeling>

The following evaluation was made in the same manner as the above-described coolness.

As the general use feeling throughout use for five minutes, the level closest to the evaluation result among the following three-level evaluation was selected, and an average value of the three panelists was calculated. The average value was rounded to an integer.
3 The cooling tool has effects on both of comfortable feeling of wear (good use feeling) and cooling sensation feeling on the head.
2 The cooling tool has any one of effects on comfortable feeling of wear (good use feeling) and cooling sensation feeling on the head.
1 The cooling tool has no effects on both of comfortable feeling of wear (good use feeling) and cooling sensation feeling on the head.

Furthermore, 400 g of water of 10 degrees centigrade was injected into and sealed in each of the prepared fluid sealed containers to serve as the cooling tool, and allowed to stand for one minute and adapted to the temperature, and each cooling tool was prepared, and then the rupture resistance when the cooling tool was dropped from the height of 1.5 m was confirmed as follows.

### <Rupture Resistance at Time of Drop from height of 1.5 m>

Each cooling tool was dropped under the following conditions, and the presence or absence of rupture was confirmed according to the following criteria 1 to 3.
· The cooling tool is squeezed so that air gaps (head spaces) except for portions which water entered are eliminated, and is firmly pinched.
· The cooling tool is dropped from the height of 1.5 m on the floor on which a stainless steel bat is placed.
· The cooling tool is dropped in direction in which both tip ends of the U-shaped cooling tool land on the floor at the same time, and if the cooling tool is inclined or only one side of the cooling tool lands earlier than the other side, the cooling tool is prepared again from the beginning. Evaluation is made with N = 1.
· The environmental temperature and humidity is 25 degrees centigrade, 50% RH.
   3 There was no damage and no water leakage occurred.
   2 The film was not damaged, but the seal portion was damaged(pin hole and/or breakage of the seal portion), and water leakage occurred.
   1 The film was cracked, the seal portion was damaged, and water leakage occurred.

These results are shown in the lower row of Table 1 below.

From these results, Examples 1 to 8 in which the glass transition temperature of the water-impermeable film forming the main body part was equal to or less than -20 degrees centigrade, the carbon dioxide gas penetration was equal to or more than 7 L/(m²·24 hr·atm) as measured under the condition of 10 degrees centigrade in accordance with JIS K 7126-2 Annex B, and the tensile strength was equal to or more than 8.38 N/10 mm as measured under the condition of 10 degrees centigrade in accordance with JIS K 7161-1 showed the fluid sealed container that was excellent in both of the followability to the human body at the low temperature and the durability at the low temperature while maintaining the carbon dioxide gas penetration at the low temperature and in which carbon dioxide gas-containing cold water is enclosed to be usable as a human body cooling tool. In addition, it was found that the cooling effects were obtained for the contact portions and the other portions. In particular, these results suggested that Examples 1 to 6 in which the fluid sealed container was formed by the film made of the thermoplastic polyurethane elastomer and having a predetermined rubber hardness of equal to or less than 85 more preferably had the cooling characteristics in which the durability at the low temperature was highly maintained and the carbon dioxide gas penetration at the low temperature and the followability on the human body at the low temperature were further increased, and Examples 1 to 3 in which the fluid sealed container was formed by the film made of the ether-based thermoplastic polyurethane elastomer were low in the predetermined permanent strain and were excellent in the followability on the human body at the low temperature and the durability at the low temperature in addition to the above-described cooling characteristics.

On the other hand, Comparative Example 1 in which the fluid sealed container was formed by the film which did not have sufficient carbon dioxide penetration at the low temperature did not exert the action of maintaining or promoting blood circulation during cooling (the cooling effects were only locally obtained), and Comparative Examples 2 and 3 in which the fluid sealed container was formed by the film having high glass transition temperature showed low followability on the human body at the low temperature and low durability at the low temperature.

**[Table 1]**

| | Example 1 | Example 2 | Example 3 | Example 4 | Example 5 | Example 6 | Example 7 | Example 8 | Comparative Example 1 | Comparative Example 2 | Comparative Example 3 |
|---|---|---|---|---|---|---|---|---|---|---|---|
| Constituent component | Urethane elastomer (Ether based) | Urethane elastomer (Ether based) | Urethane elastomer (Ether based) | Urethane elastomer (Ester based) | Urethane elastomer (Ester based) | Urethane elastomer (Ester based) | Urethane elastomer (Ester based) | Olefin (LLDPE) | Olefin (LLDPE) | Olefin (3,1-methylpentene-αOlefin copolymer) | Olefin (3,1-methylpentene-αOlefin copolymer+ SEBS) |
| Carbon dioxide gas penetration (10°C:L/(m²·24 hr·atm)) | 25 | 35 | 16 | 16 | 24 | 12 | 7 | 11 | 4 | 40 | 12 |
| Tg (°C) | -20 | -20 | -20 | -20 | -20 | -20 | -20 | -120 | -120 | 28 | 28 |
| Tm (°C) | 135 | 135 | 135 | 132 | 132 | 132 | 132 | 116 | 118 | 130 | 130 |
| Rubber hardness | 80 | 80 | 80 | 85 | 85 | 85 | 90 | 50 | 50 | 70 | 70 |
| Total thickness (µm) | 150 | 100 | 200 | 150 | 100 | 200 | 100 | 40 | 100 | 100 | 300 |
| Percentage elongation (%) when load of 0.98 N (100 q weight) is applied at 10°C | 5 | 12 | 4 | 4 | 5 | 3 | 1 | 1 | 1 | 0 | 0.1 |
| Permanent strain (%) after 100% elongation at 10°C | 8 | 8 | 8 | 13 | 15 | 13 | 15 | 76 | 72 | 92 | 87 |
| Tensile strength (N/10 mm) at 10°C | 49.2 | 24.3 | 126.7 | 60.2 | 37.8 | 105.9 | 68.1 | 8.38 | 22.9 | 21.8 | 40.3 |
| Seal strength (N/10 mm) | 15.0 | 11.2 | 17.8 | 18.0 | 18.0 | 20.0 | 17.2 | 15.1 | 15.1 | 9.0 | 19.0 |
| Skin flushing | ○ | ○ | ○ | ○ | ○ | ○ | ○ | ○ | × | ○ | ○ |
| Coolness during use of carbon dioxide gas-containing cold water of 10°C | 3 | 3 | 3 | 3 | 3 | 2 | 2 | 2 | 1 | 3 | 3 |
| Cooling sensation on head during use of carbon dioxide gas-containing cold water of 10°C | 3 | 3 | 3 | 3 | 3 | 2 | 2 | 2 | 1 | 3 | 3 |
| Fit feeling during use of carbon dioxide gas-containing cold water of 10°C | 3 | 3 | 3 | 3 | 3 | 3 | 3 | 2 | 1 | 1 | 1 |
| General use feeling during use of carbon dioxide gas-containing cold water of 10°C | 3 | 3 | 3 | 3 | 3 | 3 | 2 | 2 | 1 | 2 | 2 |
| Rupture Resistance at Time of Drop from Height of 1.5 m | 3 | 3 | 3 | 3 | 3 | 3 | 2 | 2 | 2 | 1 | 1 |

The above-described embodiments encompass the following technical ideas.
<1> A fluid sealed container comprising a main body part formed of a film made of a thermoplastic elastomer or a thermoplastic resin, and an injection port part that is connected to the main body part and is configured to be openable and closable to be able to hermetically seal the main body part, wherein
   the film forming the main body part is water-impermeable, has a glass transition temperature of less than 0 degrees centigrade, and has a carbon dioxide gas penetration of equal to or more than 5 L/(m²·24 hr·atm) as measured under the condition of 10 degrees centigrade in accordance with JIS K 7126-2 Annex B, and further has a tensile strength of equal to or more than 8 N/10 mm as measured under the condition of 10 degrees centigrade in accordance with JIS K 7161-1.
<2> The fluid sealed container according to <1>, wherein
   the film forming the main body part is a film made of a thermoplastic polyurethane elastomer.
<3> The fluid sealed container according to <2>, wherein
   the film made of a thermoplastic polyurethane elastomer has a rubber hardness of equal to or more than 50 and equal to or less than 85, more preferably equal to or more than 60 and equal to or less than 83, and further preferably equal to or more than 65 and equal to or less than 80 as measured in accordance with JIS K 6253 using a durometer of type A.
<4> The fluid sealed container according to <2> or <3>, wherein
   the thermoplastic polyurethane elastomer is an ether-based thermoplastic polyurethane elastomer.
<5> The fluid sealed container according to any one of <1> to <4>, wherein
   the film forming the main body part has a percentage elongation when a load of 0.98 N is applied as measured under a condition of 10 degrees centigrade in accordance with JIS K 7161-1, the percentage elongation being equal to or more than 2%, more preferably equal to or more than 3% and equal to or less than 20%, further preferably equal to or more than 4% and equal to or less than 18%, and still more preferably equal to or more than 4% and equal to or less than 15%.
<6> The fluid sealed container according to any one of <1> to <5>, wherein
   the film forming the main body part has a permanent strain after 100% elongation as measured under a condition of 10 degrees centigrade in accordance with JIS K 7161-1, the permanent strain being equal to or less than 20%, more preferably equal to or less than 15%, further preferably equal to or less than 13%, and still more preferably equal to or less than 10%.
<7> The fluid sealed container according to any one of <1> to <6>, wherein
   the film forming the main body part has a melting point of equal to or more than 120 degrees centigrade and equal to or less than 150 degrees centigrade, more preferably equal to or more than 125 degrees centigrade and equal to or less than 145 degrees centigrade, and further preferably more than 130 degrees centigrade and equal to or less than 140 degrees centigrade.
<8> The fluid sealed container according to any one of <1> to <7>, wherein
   the film forming the main body part has a total thickness of equal to or more than 50 µm and less than 250 µm, more preferably equal to or more than 80 µm and equal to or less than 230 µm, and further preferably equal to or more than 100 µm and equal to or less than 200 µm.
<9> The fluid sealed container according to any one of <1> to <8>, wherein
   the glass transition temperature of the film forming the main body part is equal to or less than -5 degrees centigrade, more preferably equal to or more than -90 degrees centigrade and equal to or less than -10 degrees centigrade, further preferably equal to or more than -60 degrees centigrade and equal to or less than -15 degrees centigrade, and still more preferably equal to or more than -40 degrees centigrade and equal to or less than -18 degrees centigrade.
<10> The fluid sealed container according to any one of <1> to <9>, wherein
   the carbon dioxide gas penetration of the film forming the main body part is equal to or more than 10 L/(m²·24 hr·atm), more preferably equal to or more than 12 L/(m²·24 hr·atm), further preferably equal to or more than 15 L/ (m²·24 hr·atm) and equal to or less than 100 L/ (m²·24 hr·atm), still more preferably equal to or more than 18 L/ (m²·24 hr·atm) and equal to or less than 80 L/ (m²·24 hr·atm), and still further preferably equal to or more than 20 L/ (m²·24 hr·atm) and equal to or less than 50 L/ (m²·24 hr·atm) .
<11> The fluid sealed container according to any one of <1> to <10>, wherein
   the tensile strength of the film forming the main body part is equal to or more than 10 N/10 mm, more preferably equal to or more than 15 N/10 mm and equal to or less than 150 N/10 mm, further preferably equal to or more than 20 N/10 mm and equal to or less than 130 N/10 mm, still more preferably equal to or more than 30 N/10 mm and equal to or less than 100 N/10 mm, and still further preferably equal to or more than 40 N/10 mm and equal to or less than 80 N/10 mm.
<12> The fluid sealed container according to any one of <1> to <11>, wherein
   the glass transition temperature of the film forming the main body part is equal to or more than -40 degrees centigrade and equal to or less than -18 degrees centigrade, the carbon dioxide gas penetration is equal to or more than 15 L/(m²·24 hr·atm) and equal to or less than 50 L/(m²·24 hr·atm), and further the tensile strength is equal to or more than 20 N/10 mm and equal to or less than 130 N/10 mm.
<13> A cooling tool in which carbon dioxide gas-containing cold water having a temperature of equal to or less than 15 degrees centigrade and a carbon dioxide gas concentration of equal to or more than 300 ppm is enclosed in the fluid sealed container according to any one of <1> to <12>.
<14> A method of using the cooling tool (method of cooling a human body) according to <13>, the cooling tool being used (cooled) by contacting a portion of a human body of a user, and more preferably by contacting a portion of a leg or a neck.
<15> The method of using the cooling tool according to <14>, wherein
   the cooling tool is used by directly contacting a skin of a portion of the human body of the user.

This application is based upon and claims the benefit of priority from Japanese Patent Application No. 2023-102974, filed on Jun. 23, 2023, the disclosure of which is incorporated herein in its entirety by reference.

### REFERENCE SIGNS LIST

- 100: Fluid sealed container
- 101: Cooling tool
- 21: Injection port part
- 31: Main body part
- 31-1: Branch portion

## Claims

1. A fluid sealed container comprising a main body part formed of a film made of a thermoplastic elastomer or a thermoplastic resin, and an injection port part that is connected to the main body part and is configured to be openable and closable to be able to hermetically seal the main body part, wherein
the film forming the main body part is water-impermeable, has a glass transition temperature of less than 0 degrees centigrade, and has a carbon dioxide gas penetration of equal to or more than 5 L/(m²·24 hr·atm) as measured under the condition of 10 degrees centigrade in accordance with JIS K 7126-2 Annex B, and further has a tensile strength of equal to or more than 8 N/10 mm as measured under the condition of 10 degrees centigrade in accordance with JIS K 7161-1.

2. The fluid sealed container according to claim 1, wherein
the film forming the main body part is a film made of a thermoplastic polyurethane elastomer.

3. The fluid sealed container according to claim 2, wherein
the film made of a thermoplastic polyurethane elastomer has a rubber hardness of equal to or more than 50 and equal to or less than 85 as measured in accordance with JIS K 6253 using a durometer of type A.

4. The fluid sealed container according to claim 2 or 3, wherein
the thermoplastic polyurethane elastomer is an ether-based thermoplastic polyurethane elastomer.

5. The fluid sealed container according to any one of claims 1 to 4, wherein
the film forming the main body part has a percentage elongation when a load of 0.98 N is applied as measured under a condition of 10 degrees centigrade in accordance with JIS K 7161-1 of equal to or more than 2%.

6. The fluid sealed container according to any one of claims 1 to 5, wherein
the film forming the main body part has a permanent strain after 100% elongation as measured under a condition of 10 degrees centigrade in accordance with JIS K 7161-1 of equal to or less than 20%.

7. The fluid sealed container according to any one of claims 1 to 6, wherein
the film forming the main body part has a melting point of equal to or more than 120 degrees centigrade and equal to or less than 150 degrees centigrade.

8. The fluid sealed container according to any one of claims 1 to 7, wherein
the film forming the main body part has a total thickness of equal to or more than 50 µm and less than 250 µm.

9. A cooling tool in which carbon dioxide gas-containing cold water having a temperature of equal to or less than 15 degrees centigrade and a carbon dioxide gas concentration of equal to or more than 300 ppm is enclosed in the fluid sealed container according to any one of claims 1 to 8.
